# EUROPÄISCHE PATENTSCHRIFT

(11) **EP 2 770 361 B1**
(45) Veröffentlichungstag und Bekanntmachung des Hinweises auf die Patenterteilung: **25.04.2018**
(21) Anmeldenummer: 14155389.1
(22) Anmeldetag: 17.02.2014
(51) Int. Cl.: G02B 23/24, A61B 1/00

(54) **Optikrohr für ein Endoskop, Endoskop sowie Verfahren zum Montieren mindestens einer Stablinse in einem Optikrohr**
Optical tube for an endoscope, endoscope and method for mounting at least one rod lens in an optical tube
Tube optique pour un endoscope, endoscope et procédé de montage d'au moins une lentille cylindrique dans un tube optique

(30) Priorität: 20.02.2013 DE 102013101650
(43) Veröffentlichungstag der Anmeldung: 27.08.2014
(73) Patentinhaber: Karl Storz SE & Co. KG, 78532 Tuttlingen (DE)
(72) Erfinder: Dahmen, Jan, 78532 Tuttlingen (DE); Vogel, Walter, 78532 Tuttlingen (DE)

(56) Entgegenhaltungen:
- EP-A1- 2 283 768
- EP-A2- 1 776 918
- WO-A1-99/05959
- DE-A1- 3 431 631
- DE-A1- 19 742 454
- DE-A1- 19 750 685
- DE-A1-102006 041 920
- DE-U1- 9 417 262
- US-B2- 7 708 689

## Beschreibung

Die vorliegende Erfindung betrifft ein Optikrohr für ein Endoskop nach dem Oberbegriff von Anspruch 1 sowie ein Endoskop mit einem derartigen Optikrohr und ein Verfahren zum Montieren mindestens einer Stablinse in einem Optikrohr.

Endoskope dienen zur Betrachtung von Hohlräumen im menschlichen oder tierischen Körper sowie in technischen Gegenständen. Ein Endoskop umfasst typischerweise einen lang erstreckten Endoskopschaft, der zur Einführung in den zu betrachtenden Hohlraum geeignet ist, sowie einen Kopf, der Anschlüsse und Bedienelemente sowie einen Okulareinblick aufweisen kann. Innerhalb des Endoskopschafts und des Kopfs ist ein optisches System zur Weiterleitung eines endoskopischen Bildes vom distalen, d.h. beobachterfernen, Ende des Endoskops zum proximalen, d.h. beobachternahen, Ende angeordnet. Das optische System umfasst hierfür insbesondere ein distal angeordnetes Objektiv zur Aufnahme des endoskopischen Bilds, einen Bildweiterleiter und ein am proximalen Ende des Endoskops angeordnetes Okular mit einem Okulareinblick zur visuellen Betrachtung des weitergeleiteten endoskopischen Bildes. Das Endoskop kann insbesondere als starres Endoskop mit einem starren Endoskopschaft ausgebildet sein und als Bildweiterleiter eine Stablinsenanordnung aufweisen, die in einem innerhalb des Endoskops angeordneten Optikrohr aufgenommen ist. Ferner kann das Endoskop zur Beleuchtung des zu beobachtenden Hohlraums eine Beleuchtungseinrichtung aufweisen sowie ggf. weitere Vorrichtungen, wie etwa Kanäle für endoskopische Arbeitsinstrumente.

Zur Montage der optischen Bauelemente in dem Optikrohr eines starren Endoskops werden diese üblicherweise von der proximalen Seite her in der durch die Berechnung des optischen Systems vorgegebenen Reihenfolge und Anordnung in das Optikrohr eingeschoben. Insbesondere werden nacheinander das Objektiv, eine oder mehrere Stablinsen und eine oder mehrere Aperturblenden bzw. Abstandshalter in das Optikrohr eingeschoben. Hierfür weisen die optischen Bauelemente einen Außendurchmesser auf, der geringfügig kleiner ist als der Innendurchmesser des Optikrohrs. Mit Hilfe einer Systemfeder und einer Systemabschlussführung wird das gesamte optische System in distaler Richtung vorgespannt und dadurch gegen einen distalen Abschluss des Optikrohrs, der insbesondere durch ein Deckglas gegeben sein kann, vorgespannt. Hierdurch werden die optischen Elemente in Längsrichtung an ihrer aufgrund der Berechnung des optischen Systems vorbestimmten jeweiligen Position gehalten.

Endoskope sind bei ihrer Benutzung erheblichen mechanischen und thermischen Belastungen ausgesetzt. So können bei der Handhabung Beschleunigungen bzw. Erschütterungen oder Stöße auf das Endoskop einwirken, oder der Endoskopschaft kann bei unachtsamer Handhabung einer Biegung ausgesetzt sein. Beschleunigungen und insbesondere Erschütterungen können zu Bewegungen der Linsen und der anderen optischen Bauelemente relativ zueinander bzw. relativ zum Optikrohr führen, wodurch Abrieb entstehen kann, der zur Entstehung von Streulicht führt. Ferner können sich die optischen Elemente gegeneinander verkippen und/oder sich relativ zueinander axial verschieben, wodurch die Qualität des weitergeleiteten endoskopischen Bilds ebenfalls beeinträchtigt werden kann. Erschütterungen und Biegungen des Endoskopschafts können zum Ausplatzen der Linsen oder sogar zum Linsenbruch führen, wobei die Stablinsen aufgrund ihrer Länge besonders gefährdet sind. Weiterhin sind medizinische Endoskope bei der Sterilisation durch Autoklavieren heißem Dampf unter erhöhtem Druck ausgesetzt, wobei Undichtigkeiten in der Abdichtung des optischen Systems ebenfalls zu einer Beeinträchtigung des endoskopischen Bilds führen können. Hierdurch können bei der Herstellung und bei der Benutzung eines Endoskops erhebliche Kosten entstehen.

Um die Stablinsen im Inneren des Optikrohrs lagegerecht und sicher zu fixieren und um zu vermeiden, dass eine Biegung des Endoskopschafts sich auf die Stablinsen überträgt, ist es aus der Offenlegungsschrift DE 19750685 A1 bekannt, dass die Stablinsen in einem definierten radialen Abstand zu der Innenseite des Optikrohrs durch zumindest zwei Zentrierhilfen positioniert werden und dass eine elastische Klebeverbindung zwischen der Außenseite der Stablinse und der Innenseite des Rohrs an zumindest zwei axial voneinander beabstandeten Stellen bewerkstelligt wird. Jede Zentrierhilfe weist Zentrierelemente auf, welche gleichmäßig um den Umfang der Stablinse verteilt sind.

Es ist Aufgabe der vorliegenden Erfindung, ein Optikrohr für ein Endoskop der genannten Art sowie ein Endoskop mit einem derartigen Optikrohr und ein Verfahren zum Montieren mindestens einer Stablinse in einem Optikrohr anzugeben, wobei auf einfache Weise und möglichst unter Reduzierung der Anzahl und der Herstellungskosten der notwendigen Bauteile eine sichere Positionierung und Fixierung der optischen Bauteile ermöglicht wird. Diese Aufgabe wird durch ein Optikrohr gemäß Anspruch 1, durch ein Endoskop gemäß Anspruch 8 sowie durch ein Verfahren gemäß Anspruch 9 gelöst.

Vorteilhafte Weiterbildungen der Erfindung ergeben sich aus den Unteransprüchen.

Ein erfindungsgemäßes Optikrohr für ein Endoskop ist vorzugsweise langerstreckt und weist in seinem Inneren eine Linsenanordnung auf, die mindestens eine Stablinse umfasst. Die Stablinse kann einteilig sein oder aus mehreren miteinander verkitteten Linsen bestehen. In der vorliegenden Anmeldung umfasst der Begriff "Stablinse" auch einen Planstab. Die Linsenanordnung umfasst insbesondere ein oder mehrere Relaislinsensysteme zum Weiterleiten eines von einem am distalen Ende des Endoskops angeordneten Objektiv aufgenommen endoskopischen Bilds zum proximalen Ende des Endoskops. Auch das Objektiv bzw. ein Abschnitt des Objektivs sowie gegebenenfalls ein Abschnitt des Okulars können im Optikrohr aufgenommen sein.

Erfindungsgemäß ist auf der mindestens einen Stablinse eine schweißbare Hülse fixiert, die die Stablinse abschnittsweise umschließt. Die Stablinse ist insbesondere zylindrisch abzentriert und die schweißbare Hülse koaxial zur Längsachse der Stablinse auf der Mantelfläche der Stablinse fixiert. Die schweißbare Hülse der mindestens einen Stablinse ist zur Fixierung der Stablinse an dem Optikrohr mit diesem verschweißt. Die Hülse weist hierfür einen Außendurchmesser auf, der geringfügig kleiner oder höchstens gleich dem Innendurchmesser des Optikrohrs ist, so dass die Stablinse mit der schweißbaren Hülse in das Optikrohr eingeführt werden kann. Vorzugsweise sind auch die Hülse und das Optikrohr jeweils zylindrisch ausgebildet.

Dadurch, dass auf der mindestens einen Stablinse eine Hülse fixiert ist, die die Stablinse abschnittsweise umschließt, und dass die Hülse mit dem Optikrohr verschweißt ist, wird eine sichere Fixierung der Stablinse an einer gewünschten Position innerhalb des Optikrohrs ermöglicht, wobei nur eine geringe Anzahl an Bauelementen benötigt wird. Auch bei Erschütterungen kann daher keine Verschiebung oder Verkippung der Stablinsen 2, 3 eintreten, so dass die Entstehung von Abrieb sowie eine Verschlechterung der Bildqualität vermieden werden können. Ferner wird hierdurch eine Fixierung der Stablinse innerhalb des Optikrohrs ohne Beeinträchtigung einer Dichtwirkung des Optikrohrs zur dampfdichten Abdichtung des im Optikrohr aufgenommenen optischen Systems ermöglicht.

Vorzugsweise sind die Hülse und das Optikrohr metallisch ausgebildet, in besonders bevorzugter Weise aus dem gleichen metallischen Material. Hierdurch wird das Verschweißen der Hülse mit dem Optikrohr erleichtert.

In vorteilhafter Weise ist die Hülse mit der Stablinse verklebt bzw. verkittet. Hierfür kann die Hülse einen Innendurchmesser aufweisen, der geringfügig größer als ein Außendurchmesser der Stablinse ist, so dass zwischen der Außenseite der Stablinse und der Innenseite der Hülse, wenn diese über die Stablinse geschoben ist, ein Klebespalt verbleibt, der zum Fixieren der Hülse an der Stablinse zumindest teilweise mit Klebstoff gefüllt wird. Hierdurch kann auf einfache und sichere Weise eine Fixierung der Hülse auf der Stablinse erreicht werden.

Weiter ist es besonders vorteilhaft, dass die Hülse die mindestens eine Stablinse auf einer Führungslänge umschließt, die nur einen Teil der gesamten Länge der Stablinse ausmacht. Vorzugsweise beträgt die Führungslänge maximal 50 % der Länge der Stablinse, besonders bevorzugt maximal ein Drittel der Länge oder ein Viertel der Länge der Stablinse. Die Stablinse ist somit nur in einem entsprechend kurzen Abschnitt von der mit dem Optikrohr verschweißten Hülse umschlossen. Hierdurch kann der weitere Vorteil erreicht werden, dass sich eine Biegung des Optikrohrs nur in geringem Maße auf die Stablinse überträgt, nämlich nur im Bereich der Führungslänge, wobei in diesem Bereich die Stablinse zusätzlich durch die Hülse gestützt wird. In den übrigen Abschnitten berührt die Stablinse aufgrund der Wandstärke der Hülse die Innenseite des Optikrohrs nicht, so dass sich eine Biegung des Optikrohrs nicht auf die Stablinse übertragen kann und die Stablinse auch im Fall von Erschütterungen nicht in Berührung mit dem Optikrohr gerät. Hierdurch kann eine Beschädigung der Stablinse durch Ausplatzen oder Linsenbruch verhindert werden.

Gemäß einer bevorzugten Ausführungsform der Erfindung ist die Hülse etwa mittig auf der Stablinse fixiert. Bei dieser Ausführungsform ragen distal- und proximalseitig die Enden der Stablinse über die Hülse und die durch diese definierte Führungslänge hinaus und halten in den über die Hülse hinausragenden Abschnitten entsprechend der Wandstärke der Hülse einen Abstand zur Innenwand des Optikrohrs ein. Hierdurch kann auf einfache Weise eine sichere Fixierung der Stablinse erreicht werden und eine Berührung des Optikrohrs durch die Stablinse außerhalb der relativ kurzen Führungslänge der Hülse vermieden werden.

Gemäß einer weiteren bevorzugten Ausführungsform der Erfindung umfasst die in dem Optikrohr aufgenommene Linsenanordnung mindestens eine weitere Stablinse, die der mindestens einen Stablinse benachbart ist, d.h. im Lichtweg auf diese folgt oder dieser vorangeht. Die Hülse umschließt die einander zugewandten Endabschnitte der beiden benachbarten Stablinsen und ist auf diesen insbesondere durch Kleben fixiert. Die Hülse umschließt somit die beiden Stablinsen nur abschnittsweise, so dass die Stablinsen in den übrigen Abschnitten nicht von der Hülse umschlossen ist und entsprechend der Wandstärke der Hülse von der Innenwand des Optikrohrs beabstandet sind. Bei dieser Ausführungsform werden die beiden Stablinsen durch die Hülse miteinander verbunden und relativ zueinander in einem vorbestimmten Abstand gehalten. Für die beiden Stablinsen wird somit nur eine einzige Hülse benötigt. Die Einheit aus den beiden Stablinsen und der Hülse kann als Ganzes in das Optikrohr eingeführt werden. Hierdurch wird die Montage des optischen Systems weiter vereinfacht und eine sichere Fixierung der beiden benachbarten Stablinsen im Optikrohr ermöglicht sowie ebenfalls die Gefahr einer Beschädigung der Stablinsen durch Biegung des Optikrohrs oder durch Erschütterungen vermindert.

Weiterhin ist es vorteilhaft, dass zwischen den beiden benachbarten, durch eine deren Endabschnitte übergreifende Hülse gemeinsam fixierten Stablinsen eine Blende angeordnet ist. Derartige, in der Regel ringförmig ausgebildete, dienen insbesondere als Aperturblenden dazu, die Apertur des weitergeleiteten endoskopischen Bildes zu begrenzen und die Einkopplung von Streulicht zu vermindern. Eine solche Blende kann gleichzeitig als Abstandshalter dienen, um den Abstand zwischen den einander zugewandten Endflächen der Stablinsen auf einen vorgegebenen Wert einzustellen. Anstelle oder zusätzlich zu der Blende kann auch ein Abstandshalter, etwa eine Abstandshülse, vorgesehen sein. Die Blende bzw. der Abstandshalter kann ein separates Bauteil sein, das zwischen den Stablinsen, über die die Hülse geschoben wird, angeordnet ist, kann aber auch fest mit der Hülse verbunden oder mit dieser einstückig ausgebildet sein. Hierdurch wird eine besonders einfache Ausführungsform erreicht, bei der die Anzahl der im Optikrohr zu montierenden Bauelemente weiter verringert ist. Insbesondere kann die Blende als Aperturblende ausgebildet sein und zwischen zwei Stablinsen angeordnet sein, die gemeinsam ein Relaislinsensystem bilden, d.h. eine Umkehrung zur Weiterleitung des endoskopischen Bildes vermitteln. Die durch die beiden Stablinsen, die Hülse und die Aperturblende gebildete Einheit kann somit ein vollständiges Relaislinsensystem darstellen, das in einfacher oder mehrfacher, jeweils identischer Ausführung in das Optikrohr eingeschoben wird. Hierdurch wird die Herstellung und Montage des Endoskops weiter vereinfacht.

In besonders vorteilhafter Weise besteht die Hülse aus einem ferromagnetischen Material oder weist zumindest ein solches Material auf. Hierdurch wird eine besonders einfache Handhabung bei der Positionierung der Hülse und der an dieser fixierten mindestens einen Stablinse innerhalb des Optikrohrs ermöglicht, indem mit Hilfe eines außerhalb des Optikrohrs angeordneten Magneten eine Magnetkraft auf die Hülse ausgeübt werden kann.

Ein erfindungsgemäßes Endoskop weist ein langerstrecktes Optikrohr auf, das innerhalb eines lang erstreckten Endoskopschafts angeordnet ist und auch in einen Kopf des Endoskops hineinreichen kann. Der Endoskopschaft ist als starrer Endoskopschaft ausgebildet, wobei eine gewisse Biegung zulässig sein kann. Das Optikrohr kann beispielsweise als Innenrohr einer Endoskopoptik, die hier ebenfalls als Endoskop bezeichnet wird, oder als Systemrohr eines Endoskops, das beispielsweise Arbeitskanäle für endoskopische Instrumente oder weitere Kanäle umfassen kann, ausgebildet sein. Das Endoskop bzw. die Endoskopoptik umfasst insbesondere auch eine Beleuchtungsoptik, die typischerweise Glasfaserlichtleiter zum Weiterleiten des von einer externen Lichtquelle erzeugten Beleuchtungslichts zum distalen Ende des Endoskops, von wo es zur Beleuchtung des zu beobachtenden Hohlraums ausgestrahlt wird, umfasst.

Bei einem erfindungsgemäßen Verfahren zum Montieren mindestens einer Stablinse im Inneren eines Optikrohrs für ein Endoskop wird in einem ersten Schritt auf einer Stablinse eine schweißbare Hülse fixiert. Die Hülse umschließt die Stablinse insbesondere nur abschnittsweise. Die Hülse kann auch zwei Stablinsen in ihren jeweiligen Endabschnitten umschließen und auf diesen fixiert sein. In einem zweiten Schritt wird die Stablinse bzw. werden die Stablinsen mit der schweißbaren Hülse in das Optikrohr eingeschoben, wofür der Außendurchmesser der schweißbaren Hülse geringfügig kleiner ist als der Innendurchmesser des Optikrohrs, so dass ein ausreichendes Spiel für ein Einschieben der Hülse in das Optikrohr besteht. In einem dritten Schritt wird die schweißbare Hülse mit dem Optikrohr verschweißt. Das Schweißen kann insbesondere als Laserschweißen ausgeführt werden, wobei ein oder mehrere Schweißpunkte oder Schweißlinien erzeugt werden können. Mit dem erfindungsgemäßen Verfahren wird auf besonders einfache Weise eine sichere Montage und Fixierung der mindestens einen Stablinse im Innern des Optikrohrs ermöglicht. Das Optikrohr mit der darin aufgenommenen Linsenanordnung kann bei der Montage eines Endoskops in den Endoskopschaft eingeschoben und darin fixiert werden. Die Hülse weist ein ferromagnetisches Material auf und wird unter Einsatz einer Magnetkraft, die von einem außerhalb des Optikrohrs angeordneten Magneten ausgeübt wird, in eine vorgegebene Position gebracht und in dieser Position mit dem Optikrohr verschweißt. Damit der außerhalb des Optikrohrs platzierte Magnet eine entsprechende Magnetkraft auf die Hülse ausüben kann, ist das Optikrohr vorzugsweise aus einem nicht-magnetischen Material angefertigt oder hinreichend dünnwandig ausgeführt, so dass die magnetische Kraft durch die Wandung des Optikrohrs hindurch wirken kann; vorzugsweise besteht das Optikrohr aus Edelstahl. Hierdurch wird auf besonders einfache Weise eine Positionierung des Hülse und damit der mit dieser verbundenen mindestens einen Stablinse ermöglicht.

Weiterhin ist es bevorzugt, dass die schweißbare Hülse mit Hilfe einer rohrförmigen Lehre, die als Vorschubdorn wirkt, positioniert und dabei von einer von dem äußeren Magneten ausgeübten magnetischen Zugkraft gegen eine Stirnfläche der Lehre gehalten wird. Hierdurch kann die Hülse besonders sicher in eine vorgegebene axiale Position gebracht und in dieser für den Schweißvorgang gehalten werden. Um die Hülse an der Stirnseite der Lehre zu halten, ist der äußere Magnet insbesondere axialsymmetrisch aufgebaut und umschließt das Optikrohr.

In besonders vorteilhafter Weise wird die Hülse von einem außerhalb des Optikrohrs angeordneten Magneten an die Innenseite des Optikrohrs angedrückt und sodann in der hierdurch definierten Lage mit dem Optikrohr verschweißt. Auf diese Weise kann ein zum Einführen der Hülse in das Optikrohr notwendiges Spiel zwischen der Außenseite der Hülse und der Innenseite des Optikrohrs ausgeglichen werden, indem die Hülse von der wirkenden magnetischen Kraft definiert auf eine Seite des Optikrohrs gezogen wird. Ein derartiger Montageschritt wird insbesondere für alle Stablinsen der Linsenanordnung des Endoskops durchgeführt, so dass die optischen Achsen der Stablinsen selbst dann, wenn diese nicht mit der Mittelachse des Optikrohrs übereinstimmen, in einer gemeinsamen Flucht liegen. Hierdurch wird eine sichere Fixierung der Stablinsen in einer gemeinsamen optischen Achse ermöglicht.

Gemäß einer weiteren bevorzugten Ausführungsform des Verfahrens wird das Optikrohr in eine V-förmige Nut einer als Auflage dienenden Montagevorrichtung eingelegt und durch eine in Richtung auf die Nut wirkende äußere Kraft derart deformiert, dass die Hülse an einer oder mehreren umfänglich verteilten Linien an der Innenseite des Optikrohrs anliegt. Insbesondere entstehen beim Eindrücken des Optikrohrs in die V-Nut drei achsenparallele Kontaktlinien. Dies führt nicht zu einer Verkippung oder Verschiebung der Stablinse, die somit in ihrer vorbestimmten Längsposition und Ausrichtung verbleibt. In dieser Lage wird die Hülse zumindest in einem Punkt mit der Innenseite des Optikrohrs verschweißt. Ebenso ist ein Verschweißen in einer Schweißlinie möglich, die einem Abschnitt der Kontaktlinie folgt, vorzugsweise der oberen, der V-Nut gegenüberliegenden Kontaktlinie, oder mit einander gegenüberliegenden oder umfänglich verteilten Schweißlinien. Auch in diesem Fall ist es vorteilhaft, alle Linsen der Linsenanordnung des Optikrohrs in dieser Weise zu montieren, so dass die optischen Achsen der Linsen miteinander fluchten.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 einen Abschnitt eines Optikrohrs im schematischen Längsschnitt mit im Inneren des Optikrohrs aufgenommenen Stablinsen gemäß einem ersten Ausführungsbeispiel der Erfindung sowie einen Montageschritt und eine Montagevorrichtung für ein erstes erfindungsgemäßes Verfahren zum Montieren der Stablinsen im Inneren des Optikrohrs;
Fig. 2 zwei Varianten des Optikrohrs gemäß Fig. 1 sowie eine Lehre zum Positionieren der Stablinsen in Längsrichtung gemäß dem ersten Verfahren;
Fig. 3 einen Abschnitt eines Optikrohrs im schematischen Längsschnitt mit im Inneren des Optikrohrs aufgenommenen Stablinsen gemäß einem zweiten Ausführungsbeispiel der Erfindung;
Fig. 4 den Abschnitt des Optikrohrs gemäß Fig. 3 sowie einen Montageschritt eines zweiten erfindungsgemäßen Verfahrens zum Montieren der Stablinsen im Inneren des Optikrohrs;
Fig. 5 das Ausführungsbeispiel gemäß Fig. 3 und 4 in einem weiteren Schritt des zweiten Montageverfahrens;
Fig. 6 einen distalen Endbereich eines Optikrohrs mit einem Objektiv des Endoskops im schematischen Längsschnitt;
Fig. 7 einen proximalen Endbereich eines Optikrohrs mit einem Okular des Endoskops im schematischen Längsschnitt;
Fig. 8 ein Endoskop in einer teilweise aufgebrochenen Seitenansicht.

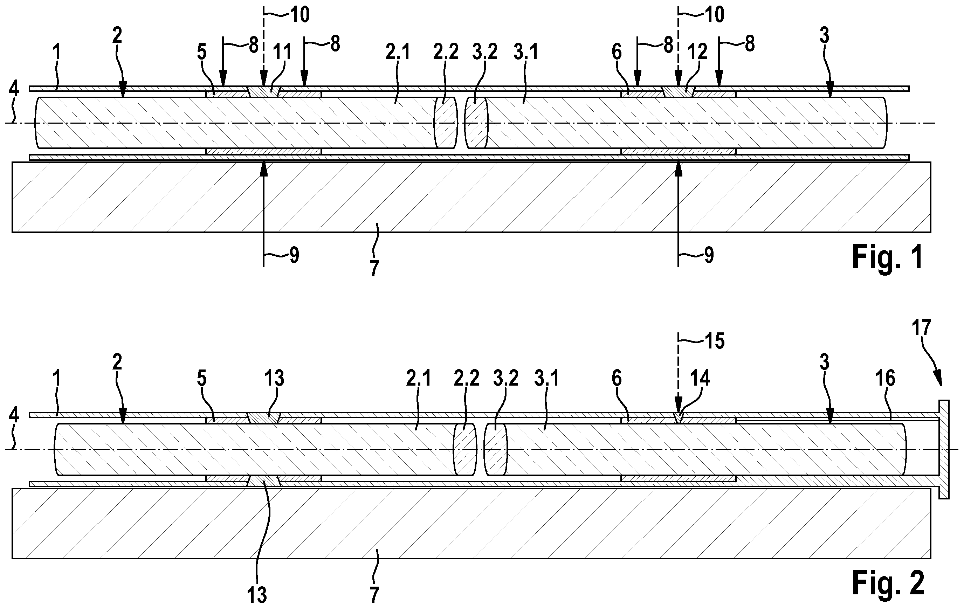

Wie in Fig. 1 dargestellt ist, sind gemäß einem ersten Ausführungsbeispiel der Erfindung in einem Optikrohr 1 eine Mehrzahl von zylindrischen Stablinsen 2, 3 angeordnet. Die Stablinsen 2, 3 bestehen beispielsweise jeweils aus einem konvex-konkaven länglichen Stabteil 2.1, 3.1, der mit einer dünnen Linse 2.2, 3.2 verkittet ist. Im dargestellten Beispiel bilden die beiden Stablinsen 2, 3 in der mit den dünnen Linsen 2.2, 3.2. einander zugewandten Anordnung ein Relaislinsensystem, das ein links von der linken Linsenfläche der linken Stablinse 2 von einem Objektiv oder einem vorgeschalteten Relaislinsensystem entworfenes Zwischenbild in ein rechts von der rechten Linsenfläche der rechten Stablinse 3 entstehendes weiteres, gegenüber dem vorigen umgekehrtes Zwischenbild abbildet. Das optische System des Endoskops umfasst je nach Länge des Endoskops in der Regel eine Mehrzahl von aufeinander folgenden Relaislinsensystemen. Die Stablinsen 2, 3 sowie die vor- und nachgeschalteten weiteren Relaislinsensysteme bzw. optischen Elemente sind im Inneren des Optikrohrs 1 derart angeordnet, dass ihre jeweiligen optischen Achsen miteinander fluchten und die optische Achse 4 des Gesamtsystems bilden.

Auf die Stablinsen 2, 3 ist jeweils eine schweißbare Hülse 5, 6 aufgeschoben und durch Kleben etwa mittig fixiert. Die Hülse 5, 6 ist zylindrisch mit einer gleichmäßigen Wandstärke ausgebildet und weist gegenüber dem Optikrohr 1 ein geringfügiges Untermaß auf, um ein Einschieben der Stablinsen 2, 3 mit ihren jeweiligen Hülsen 5, 6 in den Innenraum des Optikrohrs 1 zu ermöglichen. Um eine Verschiebung oder Verkippung der Stablinsen 5, 6 zu verhindern und diese in einer vorgegebenen Position und Ausrichtung zu halten, werden die Hülsen 5, 6 durch Schweißen mit dem Optikrohr 1 verbunden. In Fig. 1 ist, wie nachfolgend beschrieben wird, eine erste Variante einer solchen Schweißverbindung sowie ein Schritt eines ersten Montageverfahrens zum Montieren von Stablinsen im Inneren des Optikrohrs 1 dargestellt.

Gemäß dem ersten Montageverfahren wird, nachdem die Stablinsen 2, 3 mit den Hülsen 5, 6 an die vorgegebene Position innerhalb des Optikrohrs 1 geschoben worden sind, das Optikrohr 1 in eine V-förmige Nut einer als Auflage dienenden, im Wesentlichen prismatisch ausgebildeten Montagevorrichtung 7 eingelegt; dies kann auch schon vor dem Einschieben der Stablinsen 2, 3 in das Optikrohr 1 geschehen. In der durch die Pfeile 8 angedeuteten senkrechten Richtung wird sodann ein einseitiger Druck auf das Optikrohr 1 ausgeübt, durch den dieses geringfügig deformiert wird, wodurch die Innenseite des Optikrohrs 1 mit der Außenseite der Hülse 5, 6 in linienförmigen Kontakt kommt und die Stablinse 2, 3 mit der Hülse 5, 6 in der erreichten Position in achsparalleler Lage gehalten wird. Aufgrund der von den Wänden der V-förmigen Nut ausgeübten Gegenkraft, die in Fig. 1 durch die Pfeile 9 symbolisch dargestellt ist, bilden sich im unteren Bereich ein oder zwei weitere linienförmige Kontakte zwischen der Hülse 5, 6 und dem Optikrohr 1 aus. Durch eine entsprechende Wärmezufuhr von außen, etwa durch Einwirkung des Laserstrahls 10 einer Laserschweißanlage, wird nun das Material des Optikrohrs 1 und der Hülse 5, 6 genügend erwärmt, so dass eine in Längsrichtung verlaufende linienförmige Schweißnaht 11, 12 gebildet wird, durch die die Hülse 5, 6 mit dem Optikrohr fest verbunden wird. Die Schweißnaht 11, 12 braucht dabei nur einen Teil der Länge der Kontaktlinie zwischen der Hülse 5, 6 und dem Optikrohr 1 einzunehmen und kann daher eine geringere Länge haben als die Hülse 5, 6 selbst. Die mechanischen Eigenschaften des Optikrohrs 1 werden durch die Schweißverbindung nicht wesentlich beeinträchtigt; insbesondere wird das Optikrohr 1 durch den Schweißvorgang nicht durchbrochen und kann daher durch entsprechende Abdichtung am proximalen und am distalen Ende hermetisch dicht ausgeführt werden. Durch die Schweißverbindung sind die Stablinsen 2, 3 fest im Inneren des Optikrohrs 1 fixiert, so dass auch bei Erschütterungen keine Verschiebung oder Verkippung der Stablinsen 2, 3 eintreten kann.

Nach Herstellung der Schweißverbindung wird die Ausübung der seitlichen Kraft in Richtung der Pfeile 8 beendet, so dass sich das Optikrohr 1 elastisch in seine ursprüngliche zylindrische Form zurückverformt. Auch wenn die Stablinsen 2, 3 aufgrund des Untermaßes der Hülsen 5, 6 dann nicht genau zentrisch innerhalb des Optikrohrs 1 angeordnet sind, so bilden diese dennoch eine gemeinsame, ebenfalls nicht genau zentrisch innerhalb des Optikrohrs verlaufende, jedoch zur Längsachse des Optikrohrs 1 parallele optische Achse 4. Durch die Hülsen 5, 6 werden die Stablinsen 2, 3 in den freien, nicht von den Hülsen 5, 6 umschlossenen Abschnitten in einen Abstand zum Optikrohr 1 gehalten, so dass auch bei Erschütterungen sowie bei einer gewissen Biegung des Optikrohrs 1 keine Berührung zwischen dem Optikrohr 1 und den freien Bereichen der Stablinsen 2, 3 erfolgt. Hierdurch kann eine Beschädigung der Stablinsen sicher vermieden werden.

In Fig. 2 sind zwei weitere Varianten der Schweißverbindung zwischen der Hülse 5, 6 und dem Optikrohr 1 dargestellt. Im linken Teil der Fig. 2 ist angedeutet, dass mehrere in Längsrichtung des Optikrohrs 1 verlaufende Schweißnähte 13, die entlang des Umfangs der Hülse 5 angeordnet sind, zur Verbindung mit dem Optikrohr 1 vorgesehen sein können, beispielsweise zwei einander gegenüberliegende Schweißnähte 13 oder auch drei Schweißnähte an den zuvor erwähnten Kontaktlinien zwischen der Hülse 5 und dem Optikrohr 1 bei der Deformation durch die von oben einwirkende Kraft. Wie im rechten Teil der Fig. 2 gezeigt ist, kann anstelle einer linienförmigen Schweißnaht auch ein Schweißpunkt 14 zur festen Verbindung der Hülse 6 mit dem Optikrohr 1 ausreichend sein, der ebenfalls durch Einwirkung eines in Fig. 2 angedeuteten Laserstrahls 15 erzeugt werden kann. Ferner können gemäß weiterer Varianten der Schweißverbindung eine oder mehrere kurze, in Querrichtung verlaufende Schweißnähte oder insbesondere in dem Fall, dass das Untermaß der Hülse 5, 6 gegenüber dem Innenmaß des Optikrohrs 1 sehr gering ist und keine Deformation des Optikrohrs notwendig ist, eine ringförmig umlaufende Schweißnaht erzeugt werden (in Fig. 1 und 2 nicht dargestellt). Es versteht sich, dass vorzugsweise beide Stablinsen 2, 3 des in den Figuren 1 und 2 gezeigten Relaislinsensystems, insbesondere alle Relaislinsensysteme des Endoskops, mit nach der gleichen Variante ausgebildeten Schweißverbindungen im Optikrohr 1 fixiert werden.

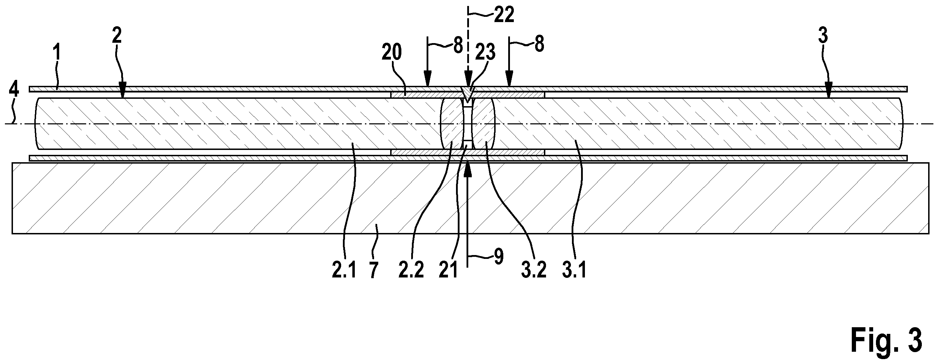

In Fig. 3 ist im Längsschnitt ein Abschnitt eines Optikrohrs mit darin angeordneten Stablinsen 2, 3 gemäß einem zweiten Ausführungsbeispiel der Erfindung dargestellt. Dabei umgreift eine Hülse 20 die einander zugewandten Endabschnitte der beiden Stablinsen 2, 3 eines Relaislinsensystems. Zwischen den Stablinsen 2, 3 ist eine ringförmige Aperturblende 21 angeordnet, die zugleich als Abstandshalter zwischen den beiden Stablinsen 2, 3 dient. Die Hülse 20 ist durch Kleben auf den jeweiligen Endabschnitten der Stablinsen 2, 3 fixiert. Durch Einschieben der Stablinsen 2, 3 in die Hülse 20 und Verkleben der Stablinsen mit der Hülse 20 wird somit eine Einheit geschaffen, die als Ganzes in das Optikrohr 1 eingeschoben werden kann. Im Übrigen ist die in Fig. 3 dargestellte Ausführungsform wie die zu den Figuren 1 und 2 beschriebene ausgebildet. Die Hülse 20 wird durch Laserschweißen mit einem Laserstrahl 22 über den Schweißpunkt 23 bzw. eine vorzugsweise quer zur Längsachse des Optikrohrs gerichtete Schweißnaht an dem Optikrohr 1 fixiert.

Die Aperturblende 21 kann, wie in Fig. 3 gezeigt, als separates Bauteil ausgebildet sein, das vor oder gemeinsam mit den Stablinsen 2, 3 in die Hülse 20 eingeführt wird. Insbesondere kann die Aperturblende ebenfalls aus einem schweißfähigen Material bestehen und beim Schweißvorgang mit der Hülse 20 verschweißt werden, wie in Fig. 3 durch den bis in die Aperturblende 21 reichenden Schweißpunkt 23 angedeutet ist. Die Aperturblende 21 kann aber auch beispielsweise durch Kleben mit der Hülse 20 verbunden werden oder einstückig mit dieser ausgebildet sein (nicht dargestellt).

In Fig. 2 ist ein erstes Verfahren zum Positionieren der Stablinsen 2, 3 in Längsrichtung des Optikrohrs 1 gezeigt. Dabei wird die Hülse 6 mit Hilfe eines Positionierungsrohrs 16 einer Lehre 17 innerhalb des Optikrohrs 1 verschoben. Das Positionierungsrohr 16 weist einen Innendurchmesser auf, der größer als der Außendurchmesser der Stablinse 3 ist, so dass diese umgriffen werden kann und die Stirnseite des Positionierungsrohrs 16 direkt an einer Stirnseite der Hülse 6 anliegt. Wie in Fig. 2 symbolisch angedeutet ist, kann die Hülse 6 mit der Lehre 17 so weit verschoben werden, bis die Lehre 17 im Anschlag mit einem proximalen oder distalen Ende des Optikrohrs 1 anliegt, wobei das Positionierungsrohr 16 eine auf die vorbestimmte Position der Stablinse 3 bzw. der Hülse 6 abgestimmte Länge hat. Es ist auch denkbar, dass die Lehre 17 mit dem Positionierungsrohr 16 nicht auf Anschlag, sondern nach Maß in das Optikrohr 1 eingeführt wird, um die Stablinse 3 in die vorbestimmte Position, die bei der Berechnung des optischen Systems ermittelt worden ist, zu schieben. In ähnlicher Weise wird für die Stablinse 2 sowie für die übrigen Linsen des optischen Systems vorgegangen. Hierdurch werden auch die vorgegebenen Luftspalte zwischen den Linsen eingestellt. Es versteht sich, dass dieses Verfahren auch bei der in Fig. 3 dargestellten Ausführungsform einsetzbar ist.

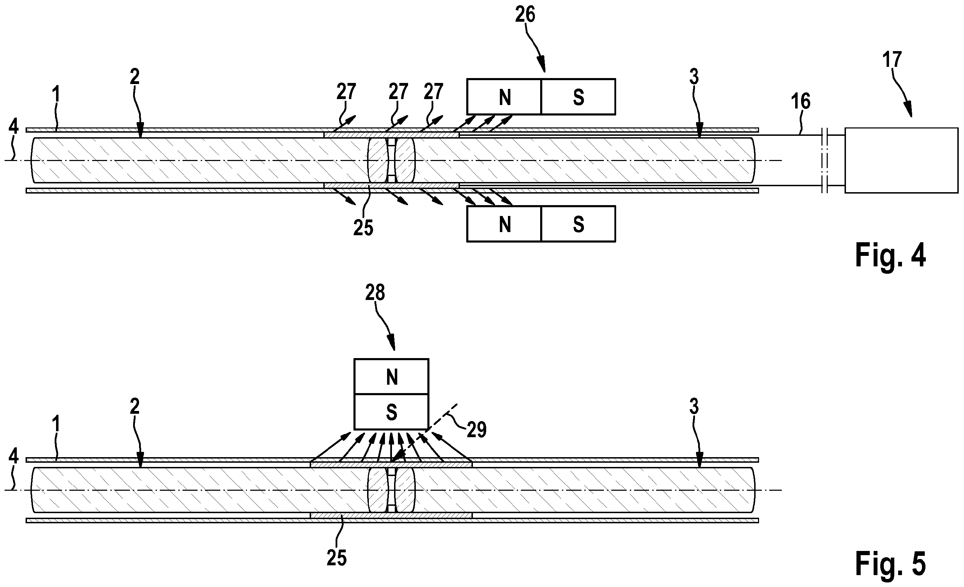

Während bei dem zuvor beschriebenen Längspositionierungsschritt die Hülse 5, 6, 20 lediglich durch Reibung in Anlage mit der Stirnfläche des Positionierungsrohrs 16 gehalten wird, kann die Anlage der Hülse am Positionierungsrohr 16 und damit die Positionierungsgenauigkeit durch das in Fig. 4 gezeigte Verfahren verbessert werden. Die Hülse 25 ist in diesem Fall aus einem ferromagnetischen Material hergestellt und wird von einem koaxial angeordneten externen Magneten 26, der gegenüber der Hülse 25 in Richtung auf die Lehre 17 hin versetzt gehalten wird, an die Stirnseite des Positionierungsrohrs 16 gezogen. Die hierdurch erzeugte Kraftwirkung ist symbolisch durch die Pfeile 27 angegeben. Insbesondere kann die Hülse aus einem magnetisch weichen ferromagnetischen Material bestehen, so dass eine beliebige Anordnung der in Fig. 4 exemplarisch eingezeichneten Pole des Magneten 26 zur Erzeugung einer entsprechenden Zugkraft geeignet ist. Das Positionierungsrohr 16 wird, wie oben erläutert, bis zu einem vorgegebenen Maß oder auf Anschlag in das Optikrohr 1 eingeschoben, so dass die Hülse 25 und die Stablinsen 2, 3 ihre vorgegebene Position in Längsrichtung des Optikrohrs 1 erreichen.

Wie in Fig. 5 gezeigt, kann die magnetische Hülse 25 durch einen seitlich des Optikrohrs 1 angeordneten Magneten 28 einseitig an die Innenseite des Optikrohrs 1 angepresst werden. Hierdurch wird ein linienförmiger Kontakt zwischen der Hülse 25 und der Innenseite des Optikrohrs 1 erzeugt, und die Stablinsen 2, 3 werden axial ausgerichtet in der erreichten Position innerhalb des Optikrohrs 1 festgehalten. Wie zuvor beschrieben, wird dann mit Hilfe eines Laserstrahls 29 eine Schweißverbindung erzeugt und die Hülse 25 mit dem Optikrohr 1 verbunden; der Magnet 28 kann eine entsprechende Aussparung für den Laserstrahl 29 aufweisen. Dabei kann je nach Untermaß der Hülse 25 gegenüber dem Optikrohr 1 ein Versatz der optischen Achse 4 gegenüber der Symmetrieachse des Optikrohrs 1 auftreten. Durch eine entsprechende Führung des Magneten 28 kann gewährleistet werden, dass die Stablinsen aller Relaislinsensysteme des Endoskops derart fixiert werden, dass ihre optischen Achsen miteinander fluchten, so dass die Qualität der optischen Abbildung durch den Versatz nicht beeinträchtigt wird. Sofern das Untermaß der Hülse 25 gegenüber dem Optikrohr 1 genügend gering ist, kann ggf. auf das seitliche Anpressen mit dem Magneten 28 verzichtet werden.

Im Übrigen ist die in den Figuren 4 und 5 dargestellte Ausführungsform wie zu Fig. 3 beschrieben ausgebildet. Es versteht sich, dass die in Figuren 4 und 5 gezeigten Montageschritte in gleicher Weise für eine gemäß Figuren 1 und 2 ausgebildete Hülse durchgeführt werden können.

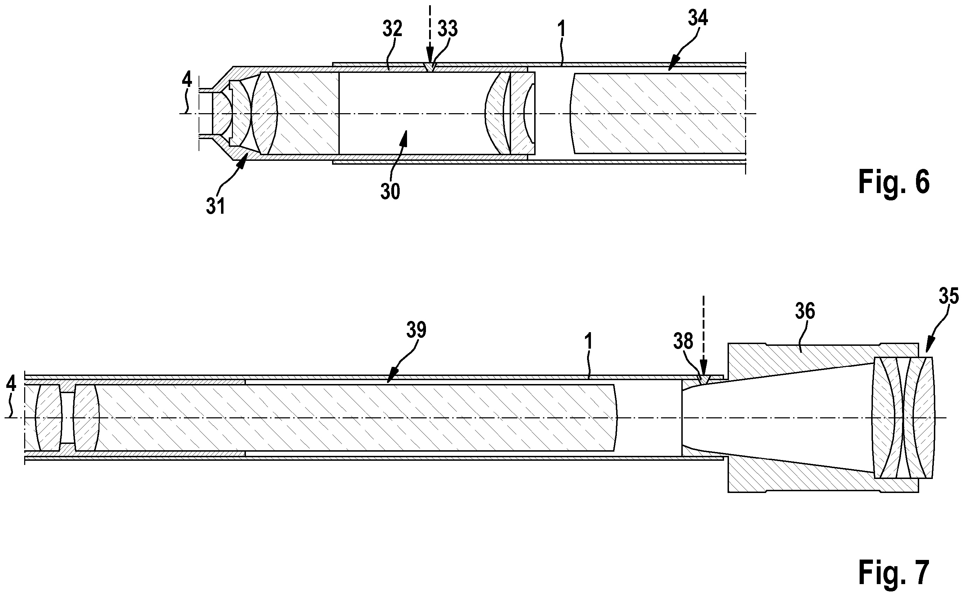

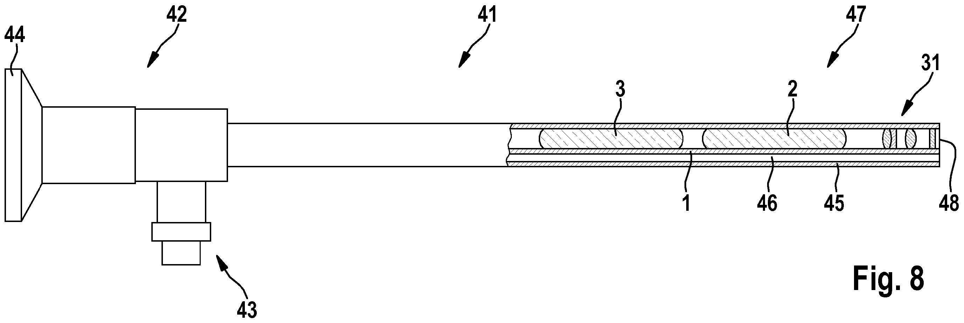

Wie in den Figuren 6 und 7 beispielhaft dargestellt, kann auch das Objektiv und/oder ein Okular des Endoskops mit dem Optikrohr verschweißt werden. Das Objektiv 30 umfasst eine Objektivlinsenanordnung 31, die in einer Objektivhülse 32 gehalten ist. Die Objektivhülse 32 ist in das Optikrohr 1 einschiebbar und mit diesem verschweißbar ausgebildet. Nach dem Einschieben in eine vorgegebene axiale Position wird die Objektivhülse 32 wie oben beschrieben durch einen oder mehrere Schweißpunkte 33 oder durch eine oder mehrere linienförmige Schweißnähte oder auch durch eine umlaufende kreisförmige Schweißnaht mit dem Optikrohr 1 verbunden. Gemäß Fig. 7 weist ein Okular eine Okularlinsenanordnung 35 auf, die in einer Okulareinheit 36 angeordnet ist, die distalseitig in einen zylindrischen Stutzen 37 übergeht, der in das proximale Ende des Optikrohrs 1 einschiebbar ist und mit diesem wie zuvor beschrieben beispielsweise über einen Schweißpunkt 38 oder eine Schweißnaht verschweißt wird. In den Figuren 6 und 7 sind innerhalb des Optikrohrs eine distalseitige Stablinse 34 und eine proximalseitige Stablinse 39 dargestellt, die wie oben beschrieben ausgebildet und innerhalb des Optikrohrs 1 fixiert sind. In Fig. 8 ist beispielhaft ein als Endoskopoptik ausgebildetes Endoskop 40 mit einem Optikrohr 1 gemäß den vorstehend beschriebenen Ausführungsbeispielen schematisch dargestellt. Das Endoskop umfasst einen langerstreckten, zur Einführung in einen körperinneren Hohlraum ausgebildeten Endoskopschaft 41 und einen am proximalen Ende des Endoskopschafts 41 angeordneten Kopf 42, der einen Lichtanschluss 43 und ein Okular mit einer Okularmuschel 44 aufweist. Innerhalb des Außenrohrs 45 sind das Optikrohr 1 sowie Lichtleiter 46 zur Übertragung des am Lichtanschluss 43 eingekoppelten Beleuchtungslichts zum distalen Endbereich 47 des Endoskops 40 angeordnet. Im Innenraum des Optikrohrs 1 sind eine Objektivlinsenanordnung 31 sowie Stablinsen 2, 3, die zur Weiterleitung des von der Objektivlinsenanordnung 31 entworfenen endoskopischen Bilds vom distalen Endbereich 47 zum proximalen Endbereich des Endoskops 40 mit dem Okular dienen, aufgenommen. Distalseitig ist das Optikrohr 1 bzw. der Endoskopschaft 41 durch ein Deckglas 48 hermetisch dicht verschlossen. Die Hülsen, die auf den Stablinsen 2, 3 fixiert und mit dem Optikrohr 1 verschweißt sind, sind in Fig. 8 nicht dargestellt.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Optikrohr
- 2: Stablinse
- 2.1: Stabteil
- 2.2: Linse
- 3: Stablinse
- 3.1: Stabteil
- 3.2: Linse
- 4: Optische Achse
- 5: Hülse
- 6: Hülse
- 7: Montagevorrichtung
- 8: Pfeil
- 9: Pfeil
- 10: Laserstrahl
- 11: Schweißnaht
- 12: Schweißnaht
- 13: Schweißnaht
- 14: Schweißpunkt
- 15: Laserstrahl
- 16: Positionierungsrohr
- 17: Lehre
- 20: Hülse
- 21: Aperturblende
- 22: Laserstrahl
- 23: Schweißpunkt
- 25: Hülse
- 26: Magnet
- 27: Pfeil
- 28: Magnet
- 29: Laserstrahl
- 30: Objektiv
- 31: Objektivlinsenanordnung
- 32: Objektivhülse
- 33: Schweißpunkt
- 34: Stablinse
- 35: Okularlinsenanordnung
- 36: Okulareinheit
- 37: Stutzen
- 38: Schweißpunkt
- 39: Stablinse
- 40: Endoskop
- 41: Endoskopschaft
- 42: Kopf
- 43: Lichtanschluss
- 44: Okularmuschel
- 45: Außenrohr
- 46: Lichtleiter
- 47: Distaler Endbereich
- 48: Deckglas

## Patentansprüche

1. Optikrohr für ein Endoskop, wobei im Inneren des Optikrohrs (1) eine Linsenanordnung aufgenommen ist, die mindestens eine Stablinse (2, 3, 34, 39) umfasst, wobei auf der mindestens einen Stablinse (2, 3, 34, 39) eine Hülse (5, 6, 20, 25) fixiert ist, die die Stablinse (2, 3, 34, 39) abschnittsweise umschließt und dass die Hülse (5, 6, 20, 25) mit dem Optikrohr (1) verschweißt ist und die Hülse (5, 6, 20, 25) die Stablinse (2, 3, 34, 39) auf einer Führungslänge umschließt, die kleiner als eine Länge der Stablinse (2, 3, 34, 39) ist, wobei die Stablinse nur in einem entsprechend kurzen Abschnitt von der mit dem Optikrohr verschweißten Hülse umschlossen ist, so dass die Stablinse in den übrigen Abschnitten aufgrund der Wandstärke der Hülse die Innenseite des Optikrohrs nicht berührt,
**dadurch gekennzeichnet, dass** die Hülse (5, 6, 20, 25) ein ferromagnetisches Material aufweist, so dass mit Hilfe eines außerhalb des Optikrohrs angeordneten Magneten eine Magnetkraft auf die Hülse ausgeübt werden kann.

2. Optikrohr nach Anspruch 1, **dadurch gekennzeichnet, dass** das Optikrohr (1) und die Hülse (5, 6, 20, 25) aus einem metallischen Material bestehen.

3. Optikrohr nach Anspruch 1 oder 2, **dadurch gekennzeichnet, dass** die Hülse (5, 6, 20, 25) mit der Stablinse (2, 3, 34, 39) verklebt ist.

4. Optikrohr nach einem der Ansprüche 1 bis 3, **dadurch gekennzeichnet, dass** die Führungslänge maximal ein Drittel der Länge der Stablinse (2, 3, 34, 39) beträgt.

5. Optikrohr nach Anspruch 4, **dadurch gekennzeichnet, dass** die Hülse (5, 6) mittig auf der Stablinse (2, 3) fixiert ist.

6. Optikrohr nach einem der Ansprüche 1 bis 3, **dadurch gekennzeichnet, dass** die Linsenanordnung mindestens eine weitere Stablinse (2, 3, 34, 39) umfasst, die der mindestens einen Stablinse (2, 3, 34, 39) benachbart ist, und dass die Hülse (20, 25) die einander zugewandten Endabschnitte der beiden benachbarten Stablinsen (2, 3, 34, 39) umschließt und auf diesen fixiert ist.

7. Optikrohr nach Anspruch **6, dadurch gekennzeichnet, dass** zwischen den beiden benachbarten Stablinsen (2, 3, 34, 39) eine Blende angeordnet ist.

8. Endoskop, **dadurch gekennzeichnet, dass** das Endoskop (40) ein Optikrohr (1) nach einem der vorhergehenden Ansprüche aufweist.

9. Verfahren zur Montage des Optikrohrs nach Anspruch 1, wobei auf der Stablinse (2, 3, 34, 39) die schweißbare Hülse (5, 6, 20, 25) fixiert wird, die Stablinse (2, 3, 34, 39) mit der schweißbaren Hülse (5, 6, 20, 25) in das Optikrohr (1) eingeschoben wird und die schweißbare Hülse (5, 6, 20, 25) mit dem Optikrohr (1) verschweißt wird.

10. Verfahren nach Anspruch 9, **dadurch gekennzeichnet, dass** die Hülse (5, 6, 20, 25) unter Einsatz einer magnetischen Kraft innerhalb des Optikrohrs (1) an einer vorgegebenen Position positioniert und in dieser Position mit dem Optikrohr (1) verschweißt wird.

11. Verfahren nach Anspruch 10, **dadurch gekennzeichnet, dass** die Hülse (5, 6, 20, 25) mit Hilfe einer Lehre (17) in axialer Richtung zur Erreichung einer Längsposition verschoben wird und dabei magnetisch gegen die Stirnfläche der Lehre (17) gehalten und in der erreichten axialen Position mit dem Optikrohr (1) verschweißt wird.

12. Verfahren nach Anspruch 10 oder 11, **dadurch gekennzeichnet, dass** die Hülse (5, 6, 20, 25) durch eine seitlich wirkende magnetische Kraft einseitig an die Innenseite des Optikrohrs (1) angedrückt und in dieser Lage mit dem Optikrohr (1) verschweißt wird.

13. Verfahren nach einem der Ansprüche 10 bis 12, **dadurch gekennzeichnet, dass** das Optikrohr (1) in eine V-förmige Nut einer Montagevorrichtung (7) eingelegt und durch eine seitlich wirkende Kraft derart deformiert wird, dass die Hülse (5, 6, 20, 25) an einer oder mehreren umfänglich verteilten Kontaktlinien an der Innenseite des Optikrohrs (1) anliegt und im Bereich mindestens eine der Kontaktlinien mit der Optikrohr (1) verschweißt wird.

## Claims

1. Optics tube for an endoscope, wherein a lens arrangement comprising at least one rod lens (2, 3, 34, 39) is received in the inside of the optics tube (1), wherein a sleeve (5, 6, 20, 25) is fixed on the at least one rod lens (2, 3, 34, 39) and partially encloses the rod lens (2, 3, 34, 39), and in that the sleeve (5, 6, 20, 25) is welded to the optics tube (1) and the sleeve (5, 6, 20, 25) encloses the rod lens (2, 3, 34, 39) along a guide length which is smaller than a length of the rod lens (2, 3, 34, 39), wherein only a suitably short portion of the rod lens is enclosed by the sleeve welded to the optics tube so that, in the other portions, the wall thickness of the sleeve means that the rod lens does not touch the inside face of the optics tube, **characterized in that** the sleeve (5, 6, 20, 25) has a ferromagnetic material so that a magnetic force can be applied to the sleeve with the aid of a magnet arranged outside the optics tube.

2. Optics tube according to Claim 1, **characterized in that** the optics tube (1) and the sleeve (5, 6, 20, 25) are made of a metallic material.

3. Optics tube according to Claim 1 or 2, **characterized in that** the sleeve (5, 6, 20, 25) is adhesively bonded to the rod lens (2, 3, 34, 39).

4. Optics tube according to one of Claims 1 to 3, **characterized in that** the guide length amounts to at most one third of the length of the rod lens (2, 3, 34, 39).

5. Optics tube according to Claim 4, **characterized in that** the sleeve (5, 6) is fixed centrally on the rod lens (2, 3).

6. Optics tube according to one of Claims 1 to 3, **characterized in that** the lens arrangement comprises at least one further rod lens (2, 3, 34, 39) which is adjacent to the at least one rod lens (2, 3, 34, 39), and **in that** the sleeve (20, 25) encloses the mutually facing end portions of the two adjacent rod lenses (2, 3, 34, 39) and is fixed thereon.

7. Optics tube according to Claim 6, **characterized in that** a diaphragm is arranged between the two adjacent rod lenses (2, 3, 34, 39).

8. Endoscope, **characterized in that** the endoscope (40) has an optics tube (1) according to one of the preceding claims.

9. Method for mounting the optics tube according to Claim 1, wherein the weldable sleeve (5, 6, 20, 25) is fixed on the rod lens (2, 3, 34, 39), the rod lens (2, 3, 34, 39) is pushed with the weldable sleeve (5, 6, 20, 25) into the optics tube (1), and the weldable sleeve (5, 6, 20, 25) is welded to the optics tube (1).

10. Method according to Claim 9, **characterized in that** the sleeve (5, 6, 20, 25) is positioned at a predefined position inside the optics tube (1) under the effect of a magnetic force and is welded to the optics tube (1) in this position.

11. Method according to Claim 10, **characterized in that** the sleeve (5, 6, 20, 25) is moved in the axial direction with the aid of a gauge (17) in order to reach a longitudinal position, in so doing is held magnetically against the front face of the gauge (17), and is welded to the optics tube (1) in the axial position reached.

12. Method according to Claim 10 or 11, **characterized in that** the sleeve (5, 6, 20, 25) is pressed in one direction onto the inside face of the optics tube (1) by a laterally acting magnetic force and is welded to the optics tube (1) in this position.

13. Method according to one of Claims 10 to 12, **characterized in that** the optics tube (1) is placed in a V-shaped groove of an assembly device (7) and, by means of a laterally acting force, is deformed in such a way that the sleeve (5, 6, 20, 25) bears at one or more circumferentially distributed contact lines on the inside face of the optics tube (1) and is welded to the optics tube (1) in the area of at least one of the contact lines.

## Revendications

1. Tube optique pour un endoscope, dans lequel un dispositif à lentilles est logé à l'intérieur du tube optique (1), lequel dispositif à lentilles comprend au moins une lentille en barre (2, 3, 34, 39) ;
dans lequel une gaine (5, 6, 20, 25) est fixée sur l'au moins une lentille en barre (2, 3, 34, 39), laquelle gaine enveloppe la lentille en barre (2, 3, 34, 39) par sections et que la gaine (5, 6, 20, 25) est soudée au tube optique (1) et la gaine (5, 6, 20, 25) enveloppe la lentille en barre (2, 3, 34, 39) sur une longueur de guidage qui est inférieure à une longueur de la lentille en barre (2, 3, 34, 39) ;
dans lequel la lentille en barre n'est enveloppée par la gaine soudée avec le tube optique que dans une section conformément courte, de telle sorte que la lentille en barre n'entre pas en contact avec la face interne du tube optique dans les autres sections en raison de l'épaisseur de la paroi de la gaine ;
**caractérisé en ce que** la gaine (5, 6, 20, 25) présente un matériau ferromagnétique, de telle sorte qu'une force magnétique peut être exercée sur la gaine à l'aide d'aimants, lesquels sont disposés à l'extérieur du tube optique.

2. Tube optique selon la revendication 1, **caractérisé en ce que** le tube optique (1) et la gaine (5, 6, 20, 25) sont constitués d'un matériau métallique.

3. Tube optique selon la revendication 1 ou 2,
**caractérisé en ce que** la gaine (5, 6, 20, 25) est encollée avec la lentille en barre (2, 3, 34, 39).

4. Tube optique selon l'une des revendications 1 à 3,
**caractérisé en ce que** la longueur maximale de guidage correspond à un tiers de la longueur de la lentille en barre (2, 3, 34, 39).

5. Tube optique selon la revendication 4, **caractérisé en ce que** la gaine (5, 6) est fixée de manière centrée sur la lentille en barre (2, 3).

6. Tube optique selon l'une des revendications 1 à 3,
**caractérisé en ce que** le dispositif à lentilles comprend au moins une autre lentille en barre (2, 3, 34, 39) qui est adjacente à l'au moins une lentille en barre (2, 3, 34, 39) et **en ce que** la gaine (20, 25) enveloppe les sections d'extrémité des deux lentilles en barre (2, 3, 34, 39) adjacentes, lesquelles sections d'extrémité se font mutuellement face, et est fixée sur celles-ci.

7. Tube optique selon la revendication 6, **caractérisé en ce qu'**un diaphragme est disposé entre les deux lentilles en barre (2, 3, 34, 39) adjacentes.

8. Endoscope, **caractérisé en ce que** l'endoscope (40) présente un tube optique (1) selon l'une des revendications précédentes.

9. Procédé destiné au montage du tube optique selon la revendication 1, dans lequel la gaine (5, 6, 20, 25) pouvant être soudée est fixée sur la lentille en barre (2, 3, 34, 39), la lentille en barre (2, 3, 34, 39) est insérée dans le tube optique (1) avec la gaine (5, 6, 20, 25) pouvant être soudée et la gaine (5, 6, 20, 25) pouvant être soudée est soudée au tube optique (1).

10. Procédé selon la revendication 9, **caractérisé en ce que** la gaine (5, 6, 20, 25) est positionnée à l'intérieur du tube optique (1) dans une position prédéfinie en recourant à une force magnétique et est soudée au tube optique (1) dans cette position.

11. Procédé selon la revendication 10, **caractérisé en ce que** la gaine (5, 6, 20, 25) est glissée dans la direction axiale à l'aide d'un gabarit (17) en vue de parvenir à une position longitudinale et, à cet effet, est maintenue magnétiquement contre la surface frontale du gabarit (17) et est soudée au tube optique (1) dans la position axiale ainsi atteinte.

12. Procédé selon la revendication 10 ou 11,
**caractérisé en ce que** la gaine (5, 6, 20, 25) est pressée unilatéralement contre la face interne du tube optique (1) par une force magnétique qui agit latéralement et est soudée avec le tube optique (1) dans cette position.

13. Procédé selon l'une des revendications 10 à 12,
**caractérisé en ce que** le tube optique (1) est mis en place dans une gorge, en forme de « V », d'un dispositif de montage (7) et est déformé par une force qui agit latéralement, de telle sorte que la gaine (5, 6, 20, 25) repose sur une ou plusieurs lignes de contact, laquelle ou lesquelles sont réparties sur la périphérie, au niveau de la face interne du tube optique (1) et est soudée avec le tube optique (1) dans la zone d'au moins l'une des lignes de contact.
